# EUROPÄISCHE PATENTSCHRIFT

(11) **EP 0 498 334 B1**
(45) Veröffentlichungstag und Bekanntmachung des Hinweises auf die Patenterteilung: **24.05.2000**
(21) Anmeldenummer: 92101709.1
(22) Anmeldetag: 03.02.1992
(51) Int. Cl.: C07D 401/14, C07D 405/14, A61K 31/44

(54) **2,4- und 2,5-Bis-tetrazolylpyridine, Verfahren zu deren Herstellung sowie deren Verwendung als Arzneimittel**
2,4- and 2,5-bis-(tetrazolyl)pyridines, process for their preparation and their use as medicines
Bis-(tétrazolyl)pyridines-2,4 et 2,5 procédé pour leur préparation et leur utilisation comme médicaments

(30) Priorität: 05.02.1991 DE 4103372
(43) Veröffentlichungstag der Anmeldung: 12.08.1992
(73) Patentinhaber: HOECHST AKTIENGESELLSCHAFT, 65926 Frankfurt am Main (DE)
(72) Erfinder: Schubert, Gerrit, W-6233 Kelkheim (Taunus) (DE); Baader, Ekkehard, W-6240 Königstein/Taunus (DE); Bickel, Martin, W-6380 Bad Homburg (DE); Günzler-Pukall, Volkmar, W-3550 Marburg (DE)

(56) Entgegenhaltungen:
- EP-A- 0 176 741
- EP-A- 0 278 453
- EP-A- 0 376 223
- EP-A- 0 409 119

## Beschreibung

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktion. In deren Verlauf wird Proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zeilen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie α,α'-Dipyridyl zu einer Hemmung der C1_{q}-Biosynthese von Makrophagen führt (W. Müller et al., FEBS Lett. 90 (1978), 218; Immunbiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- und - 2,5-dicarbonsäure effektiv gehemmt wird (K. Majamaa et al., Eur. J. Biochem. 138 (1984) 239-245). Diese Verbindungen sind in der Zellkultur allerdings nur in sehr hoher Konzentration als Hemmstoffe wirksam (Tschank, G. et al., Biochem. J. 248 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen. So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den Deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid vorgeschlagen.

Die deutsche Patentanmeldung P 39 24 093.2 schlägt neue N,N'-Bis(alkoxyalkyl)-pyridin-2,4-dicarbonsäurediamide vor.

Die deutsche Patentanmeldung P 40 01 002.3 beschreibt die Verwendung von Pyridin-2,4- und 2,5-dicarbonsäuredi-(nitroxyalkyl)amide zur Herstellung von Prolin-und Lysinhydroxylase hemmenden Arzneimitteln.

Sowohl Pyridin-2,4- und -2,5-dicarbonsäurediamid (Hirakata et al., J. pharm. Soc. Japan 77 (1957) 219 und Häring et al., Helv. 37 (1954) 147, 153), als auch Pyridin-2,4 und -2,5-dicarbonsäuredihydrazid (Itai et al., Bl. nation. hyg. Labor. Tokyo, 74 (1956) 115, 117 und Shinohara et al., Chem. High Polymers, Japan, 15 (1958) 839) sind bereits als Tuberkulosemittel bekannt.

In der JP 53/28175 (78/28175) werden N,N'-bis(2-nitrooxyethyl)pyridin-2,4- und -2,5-dicarbonsäurediamide als Substanzen mit vasodilatorischer Wirkung beschrieben.

Die deutsche Patentanmeldung P 40 20 570.3 beschreibt die Verwendung von 2,4- und 2,5-substituierten Pyridin-N-oxiden zur Herstellung von Prolin- und Lysinhydroxylase hemmenden Arzneimitteln.

Überraschend wurde nun gefunden, daß 2,4- und 2,5-Bis-tetrazolylpyridine der unten angegegebenen allgemeinen Formel I sowie deren physiologisch verträglichen Salze die Lysin- und Prolinhydroxylase im Tiermodell wirksam inhibieren.

Als 2,4- und 2,5-Bis-tetrazolylpyridine werden Verbindungen der allgemeinen Formel I genannt, 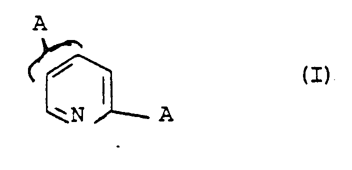 wobei A 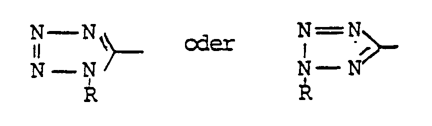 bedeutet und hierunter
- R: H,
(C₁-C₆)Alkyl, (C₂-C₆)-Alkenyl, (C₂-C₆)-Alkinyl, jeweils gegebenenfalls, bevorzugt aber für Alkyl, substituiert durch Carboxyl oder Carboxy (C₁-C₄)alkylester, durch , Phenylcarbonyl durch (C₁-C₄)Alkylaminocarbonyl, wobei Alkyl seinerseits durch (C₁-C₆)-Alkoxy, insbesondere (C₁-C₄)Alkoxy substituiert sein kann, durch Amino-Carbonyl, wobei der Stickstoff einfach, bevorzugt aber zweifach mit (C₁-C₃)Alkyl, substituiert sein kann,
(C₆-C₁₀)-Aryl, insbesondere Phenyl oder Naphthyl, gegebenenfalls substituiert durch Halogen (Chlor oder Brom), (C₁-C₆)-Alkyl oder (C₁-C₃)Alkoxy,
Phenyl-(C₂-C₆)alkyl, wobei gegebenenfalls eine oder mehrere, aber maximal 3, CH₂-Gruppen des Alkylrestes durch Heteroatome, insbesondere N, O oder S ersetzt sein können und Ar insbesondere Phenyl bedeutet,
(C₅-C₆) Cycloalkyl- oder (C₅-C₆) Cycloalkenyl-(C₀-C₆)-alkyl, gegebenenfalls mit ankodensiertem Benzolring, wobei 1 bis 3 CH₂-Gruppen des Cyclus durch Heteroatome O, S oder N, insbesondere O und/oder N, und/oder C=0 ersetzt sind und der Cyclus gegebenenfalls auch durch (C₁-C₄)-Alkyl substituiert sein kann.

Besonders bevorzugt sind Alkylreste mit 1-4 C-Atomen, Phenyl-(C₁-C₃)alkyl mit einer CH₂-Gruppe des Alkylrests ersetzt durch ein Heteroatom, insbesondere N oder O, Cycloalkyl oder Cycloalkenyl, wobei, sofern zwei CH₂-Gruppen durch jeweils eine C=0-Gruppe, eine dritte CH₂-Gruppe durch N ersetzt ist oder, sofern zwei CH₂-Gruppen durch jeweils ein O ersetzt sind, eine dritte Gruppe durch C=0 oder N ersetzt ist. Insbesondere sind die entstandenen Verbindungen achsensymmetrisch, d.h. die jeweils dritte Gruppe bindet die beiden anderen.

Insbesondere bevorzugt sind Verbindungen der Formel I, in denen die Reste R für beide Substituenten A identisch sind. Hierunter sind bevorzugt diejenigen Verbindungen zu verstehen, für die das die Substitutionsmuster für A (2,4 oder 2,5) identisch sind.

Weiterhin betrifft die Erfindung ein Verfahren zur Herstellung von Verbindungen der allgemeinen Formel I, in dem man
eine Verbindung der Formel II 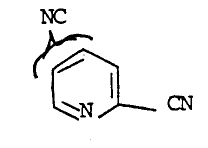 mit NaN₃ und NH₄Cl in einem geeigneten organischen Lösungsmittel umsetzt.

Sollen Verbindungen der Formel I erhalten werden, in denen R nicht H ist, so schließt sich an die genannte Reaktion noch eine Substitutionsreaktion an, in der R durch den jeweiligen Substituten ersetzt wird. Hierzu wird eine Lösung von 2,4- bzw. 2,5-Bis-(tetrazol-5-yl)-pyridin in einem geeigneten Lösungsmittel, vorzugsweise DMF oder Aceton bei Raumtemperatur mit einem Überschuß einer geeigneten Base z.B. Triethylamin oder NaOH versetzt. Anschließend wird z.B. ein Alkylierungsmittel in Substanz oder in DMF gelöst zugegeben und das Reaktionsgemisch rühren gelassen oder am Rückfluß zum Sieden erhitzt, bis dünnschichtchromatographisch (Kieselgel, Fließmittel, Ethylacetat) kein oder nur noch wenig Edukt mehr erkennbar ist. Überschüssiges Alkylierungsmittel wird eventuell durch Zugabe von konzentriertem Ammoniak zerstört. Die Aufarbeitung erfolgt entweder a) durch Eindampfen des Gemischs und blitzchromatographische Reinigung oder Umkristallisieren des Rückstands oder b) Verteilung zwischen Wasser und einem geeigneten Lösungsmittel, vorzugsweise Ethylacetat oder CH₂Cl₂, Trocknen der organischen Phasen mit Na₂SO₄, Eindampfen der Lösung und blitzchromatographische Reinigung oder Umkristallisieren des Rückstandes.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ-III oder der N- bzw. C-terminalen Quervernetzungsdomäne des Kollagens Typ-IV (7s-Kollagen bzw. Typ-IV-Kollagen-NC₁) im Serum bestimmt werden.

Zu diesem Zweck wurden die Hydroxyprolin-, Prokollagen-III-Peptid-, 7s-Kollegen- und Typ-IV-Kollagen-NC₁-Konzentration in der Leben von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde gemessen (diese Testmethode wird beschrieben von Roullier, C., experimental toxic injury of the liver; in The Liver C. Roullier, Vol. 2, S. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeignenten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,01 -25,0 mg/kg/Tag, vorzugsweise 0,01 - 5,0 mg/kg/Tag oder parenteral in Dosen von 0,001 - 5 mg/kg/Tag, vorzugsweise 0,001 -2,5 mg/kg/Tag, insbesondere 0,005- 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindugen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 % vorteilhalterweise zwischen 10 und 75% beträgt.

Geeignete Hilfs- bzw. Trägerstoffe für die gewünschte Arneimittelformulierung sind beispielsweise neben, Lösemitteln, Gelbbindern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen.

Als inerte Trägerstoffe können z.B. Gummi arabicum, Magnesia, Magnesiumcarbonat, Kaliumphoshat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z.B. in Frage physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

### 2,4-Bis-(tetrazol-5-yl)-pyridin (1)

Ein Gemischvon 100 g 2,5-Dicyanopyridin, 122.8 g NaN₃ und 12.8 g NH₄Cl wurde in 910 ml abs. DMF während 36 h auf 80-100°C erhitzt. Der gebildete beige Kristallbrei wurde mit 500 ml H₂O versetzt und anschließend unter Rühren und Eiskühlung mit konz. HCl bis pH 1 angesäuert. Der gebildete Brei wurde abgesaugt, der Rückstand in ca. 1 l 2N NaOH-Lösung unter Rühren gelöst und filtriert. Nach erneutem Ansäuern auf pH 1 mit konz. HCl wurde wiederum abgesaugt und der Rückstand im Vakuum bei 50°C getrocknet. Man erhielt 165.0 g (99 %) 1, beige Kristalle, Fp. 265-267°C (Zers.).
¹H-NMR (60 MHz, DMSO-d6)
δ=8.1 (dd, J=6Hz, J'=2Hz, 1H), 8.8 (s, 1H), 9.0 (d, J=5Hz, 1H), 7-10 (bs, 2H) ppm.
C₇H₆N₉ Ber. 216 Gef. 216 (M⁺+H⁺)

### 2,5-Bis-(tetrazol-5-yl)-pyridin (2)

230 mg 2,5-Dicyanopyridin, 283 mg NaN₃ und 36 mg NH₄Cl wurden in 3 ml abs. DMF suspendiert und 36 h bei 80-100°C gerührt. Nach dem Abkühlen wurde mit H₂O verdünnt, über Amberlite IR-120 (H⁺-Form) filtriert und mit H₂O nachgespült. Das Filtrat wurde im Vakuum eingedampft: 348 mg (91 %), 2 farblose Kristalle, Fp. ≻200°C.
¹H-NMR (60 MHz, DMSO-d6)
δ=5.5-8.0 (bs), 8.6 (m, 2H), 9.5 (s, 1H) ppm.
C₇H₆N₉ Ber. 216 Gef. 216 (M⁺+H⁺)

### Isomere 2,4-Bis-(methyltetrazol-5-yl)-pyridine (3a, b)

Zu einer Suspension von 1.2 g 2,4-Bis-(tetrazol-5-yl)-pyridin in 15 ml Aceton wurden bei Raumtemperatur 0,69 ml (11.2 mmol) Methyliodid mit der Spritze zugegeben. Anschließend wurde soviel 2N NaOH-Lösung zugegeben, bis eine klare Lösung entstanden war (ca. 6 ml) und die Lösung unter Rühren am Rückfluß zum Sieden erhitzt. Nach 2 h wurden nochmals 0.69 ml Methyliodid zugetropft. Nach insgesamt 5 h Reaktionszeit wurde abgekühlt und 10 ml konz. Ammoniak-Lösung zugegeben. Das Gemisch wurde mehrfach mit CH₂Cl₂ extrahiert, die organischen Phasen getrocknet und im Vakuum eingedampft. Man erhielt 2.21 g eines Öls, das über eine Kieselgelsäule (200 g) mit Ethylacetat als Eluens gereinigt wurde.
0.74 g 3a, farblose Kristalle vom Fp. 198-198.5°C (Ethylacetat/Cyclohexan),
¹H-NMR (60 MHz, CDCl₃)
δ=4.5 (s, 3H), 4,6 (s, 3H), 8.2 (dd, J=5 Hz, J'=2Hz, 1H), 8.9 d (J=5Hz, 1H), 9.1 (s, 1H) ppm.
C₉H₁₀N₉ Ber. 244 Gef. 244 (M⁺+H⁺)
sowie 0.29 g 3b, farblose Kristalle vom Fp. 214-215°C (Ethylacetat/Cyclohexan),
¹H-NMR (60 MHz, CDCl₃)
δ=4.5 (s, 6H), 8.2 (dd, J=5Hz, J'=2Hz, 1H), 8.95 (d, J=4Hz, 1H) überlagert mit: 9.0 (s, 1H) ppm.
C₉H₁₀N₉ Ber. 244 Gef. 244 (M⁺+H⁺)

### 2,5-Bis-(2-methyl-tetrazol-5-yl)-pyridin (4)

Zu einer Lösung von 1.5 g 2,4-Bis-(tetrazol-5-yl)-pyridin und 1.4 ml Triethylamin in 7 ml abs. DMF wurden mit der Spritze 0.85 ml CH₃l zugegeben und das Gemisch während 8 h unter Rühren am Rückfluß zum Sieden erhitzt. Nach dem Abkühlen wurden 10 ml konz. Ammoniak-Lösung zugegeben. Das Gemisch wurde mehrfach mit Ethylacetat extrahiert, die organischen Phasen getrocknet und im Vakuum eingedampft. Das zurückgebliebene Öl wurde an einer Kieselgelsäule (200 g) mit Heptan/Ethylacetat 1:1 als Eluens gereinigt. Farblose Nadeln, Fp. 225-226°C.
¹H-NMR (DMSO-d6, 60 MHz)
δ=4,5 (s, 3H), 4.55 (s, 3H), 8.6 (m, 2H), 9.5 (s, 1H) ppm.
C₉H₁₀N₉ Ber. 244 Gef. 244 (M⁺+H⁺)

### Isomere 2,4-Bis-(phenacyltetrazol-5-yl)-pyridine (5a, b)

Zu einer Suspension von 1.5 g 2,4-Bis-(tetrazol-5-yl)-pyridin in 7 ml abs. DMF wurden mitder Spritze 1.4 ml NEt₃ zugetropft. Zu der nun klaren Lösung wurde unter Rühren bei Raumtemperatur 2,78 g Phenacylbromid auf einmal zugefügt. Nach wenigen Minuten trübte sich die Lösung. Nach 2 h wurde Ethanol zugegeben, filtriert und das Filtrat im Vakuum eingedampft. Das zurückgebliebene gelbe Öl wurde über eine Kieselgelsäule (200 g) mit Ethylacetat/Heptan 2:1 als Eluens chromatographiert: 541 mg 5a, farblose, feine Nadeln, Fp: 164-165°C (Ethylacetat),
¹H-NMR (60 MHz, CDCl₃)
δ=6.2 (s, 1H), 6.5 (s, 2H), 7.7 (m, 6H), 8.0-8.3 (sh, 5H), 8.6 (d, J=6Hz, 1H), 9.2 (s, 1H) ppm.
C₂₃H₁₈N₉O₂ Ber. 452 Gef. 452 (M⁺+H⁺)
611 mg 5b, farbloses Kristallpulver, Fp. 188-189°C (Zers.).

¹H-NMR (60 MHz, CDCl₃)
δ=6.3 (s, 6H), 7.5 (m, 6H), 7.9-8.3 (sh, 3H), 8.8-9.2 (m, 2H) ppm.
C₂₃H₁₈N₉O₂ Ber. 452 Gef. 452 (M⁺+H⁺)

### 2,4-Bis-[2-(N-2-methoxyethyl-acetamido)-tetrazol-5-yl]-pyridin (6)

### Chlorameisensäure-2-methoxyethylamid

Zu einem Gemisch aus 30.09 g 2-Methoxyethylamin, 100 g 20-prozentiger wäßriger NaOH-Lösung und 150 ml 1,2-Dichlorethan wurden unter starkem Rühren und Kühlung auf -10 bis -15°C 39.7 ml Chloracetylchlorid zugetropft. Nach 1 h wurden die Phasen getrennt und die wäßrige Phase zweimal mit CH₂Cl₂ extrahiert. Die vereinigten organischen Phasen wurden nacheinander mit 2N HCl, gesätt. NaHCO₃-Lösung und H₂O gewaschen und die organischen Phasen getrocknet. Nach dem Abdampfen des Lösungsmittels blieben 60.3 g farbloses Öl zurück, das ohne weitere Reinigung weiterverwendet wurde.

Eine Lösung von 1.5 g 2,4-Bis-(tetrazol-5-yl)-pyridin, 1.4 ml Triethylamin und 2.12 g Chloressigsäure-2-methoxyethylamid in 7 ml DMF wurde unter Zugabe einer Spatelspitze Kl 48 h lang auf 80°C erhitzt. Anschließend wurde abgekühlt, mit Ethylacetat verdünnt und filtriert. Beim Einengen des Filtrats kristallisierten 1.46 g 6, farblose Kristalle, Fp. 192-194°C.
¹H-NMR (60 MHz, DMSO-d6)
δ=3.3 (sh, 14H), 5.6 (bs, 4H), 8.1-9.1 (sh, 5H) ppm.
C₁₇H₂₄N₁₁O₄ Ber. 446 Gef. 446 (M⁺+H⁺)

### 2,5-Bis-[2-(N-2-methoxyethyl-acetamido)-tetrazol-5-yl]-pyridin (7)

wurdeanalog2,4-Bis-[2-(N-2-methoxyethyl-acetamido)-tetrazol-5-yl]-pyridinhergestellt. Farbloses Kristallpulver, Fp. 250°C (Zers.).
¹H-NMR (DMSO-d6, 60 MHz)
δ=3.1-3.5 (sh, 14H), 5.65 (s, 4H), 8.3-9.0 (sh, 4H), 9.45 (m, 1H) ppm.
C₁₇H₂₄N₁₁O₄ Ber. 446 Gef. 446 (M⁺+H⁺)

### 2,4-Bis-[2-(phtalimido-N-methyl)-tetrazol-5-yl]-pyridin (8)

Eine Lösung von 1 g 2,4-Bis-(tetrazol-5-yl)-pyridin, 1 ml Triethylamin und 1.63 g Chlormethylphthalimid in 4.6 ml abs. DMF wurde bei Raumtemperatur 48 h gerührt, anschließend eingedampft und der Rückstand über eine Kieselgelsäule (200 g) mit Ethylacetat/Heptan 1:1 als Eluens gereinigt: 100 mg 8, farblose Kristalle vom Fp. 224-226°C (Zers.).
¹H-NMR (60 MHz, CDCl₃)
δ=5.6 (s, 4H), 7.0 (s, 4H), 7.7-8.2 (sh, 8H), 8.3 (dd, J=5Hz, J'=2Hz), 9.0 (d, J=6Hz, 1H), 9.1 (s, 1H) ppm.
C₂₅H₁₆N₁₁O₄ Ber. 534 Gef. 534 (M⁺+H⁺)

### 2,4-Bis-[2-(2,5-dioxopyrrolidon-1-methyl)-tetrazol-5-yl]-pyridin (9)

Eine Lösung von 0.94 g 2,4-Bis-(tetrazol-5-yl)-pyridin 1.7 ml Triethylamin und 1.3 g N-Chlormethylsuccinimid in 10 ml abs. DMF wurde 24 h bei Raumtemperatur gerührt. Anschließend wurde das Gemisch zwischen Ethylacetat und H₂O verteilt, die organischen Phasen getrocknet und eingedampft. Es verblieben 2.05 g eines Öls, das über eine Kieselgelsäule (40 g) mit Ethylacetat als Eluens gereinigt wurde. Man erhielt 0.72 g 9, farblose Kristalle vom Fp. 190-192°C.
¹H-NMR (60 MHz, DMSO-d6)
δ=2.6 (s, 4H), 2.7 (s, 4H), 6.3 (s, 2H), 6.6 (s, 2H), 8.2 (dd, J=5Hz, J'=2Hz, 1H), 8.7 (s, 1H), 9.1 (d, J=6Hz, 1H) ppm.
C₁₇H₁₆N₁₁O₄ Ber. 438 Gef. 438 (M⁺+H⁺)

### 2,4-Bis-[2-(N,N-dimethylcarbamoyl)-tetrazol-5-yl)-pyridin (10)

Eine Lösung von 0.5 g 2,4-Bis-(tetrazol-5-yl), 1.6 ml Triethylamin und 0.43 ml Dimethylcarbamoylchlorid in 5 ml abs. DMF wurde während 24 h bei Raumtemperatur gerührt, überschüssiges Dimethylcarbamoylchlorid mit Methanol zerstört und das Gemisch anschließend im Vakuum eingeengt. Man erhielt 1.86 g eines Öls, das über eine Kieselgelsäule (40 g) mit CH₂Cl₂/Methanol, Gradient 20:1 bis 9:1 als Eluens gereinigt wurde. Man erhielt 0.38 g 10, farblose Kristalle vom Fp. 150°C (Zers.).
¹H-NMR (60 MHz, CDCl₃)
δ=3.2 (s, 6H), 3.3 (s, 6H), 8.3 (dd, J=5Hz, J'=2Hz, 1H), 9.0-9.2 (sh, 2H) ppm.
C₁₃H₁₆N₁₁O₂ Ber. 358 Gef. 353 (M⁺+H⁺)

### 2,4-Bis-[2-(4-methyl-1,3-dioxol-2-on-5-yl)-methyl]-tetrazol-5-yl]-pyridin (11)

Eine Lösung von 1.0 g 2,4-Bis-(tetrazol-5-yl)-pyridin, 1.8 ml Triethylamin und 1.83 g 5-Brommethyl-1,3-dioxol-4-methyl-2-on in 10 ml abs. DMF wurden während 5 h bei Raumtemperatur gerührt und anschließend im Vakuum eingedampft. Der Rückstand wurde über eine Kieselgelsäule (160 g) mit n-Heptan/Etyhlacetat, Gradient 1:1 bis 1:3 als Eluens gereinigt. Man erhielt 0.24 g 11, farblose Kristalle, Fp. 150-152°C (Pentan/CH₂Cl₂).
¹H-NMR (60 MHz, CDCl₃)
δ=2.2 (s, 3H), 2.3 (s, 3H), 5.7 (s, 2H), 6.15 (s, 2H), 8.3 (d, J=5Hz, 1H), 8.9 (d, J=6Hz, 1H), 9.2 (s, 1H) ppm.
C₁₇H₁₃N₉O₆ Ber. 440 Gef. 440 (M⁺+H⁺)

### Isomere 2,4-Bis-(ethoxycarbonylmethyl-tetrazol-5-yl)-pyridine (12a, b)

Eine Lösung von 1,5 g Bis-(tetrazol-5-yl)-pyridin,
2.7 ml Triethylamin und 1.65 ml Iodessigsäureethylester in 10 ml DMF wurde während 3 h bei 60°C gerührt. Nach Reaktionskontrolle mittels DC wurden weitere 0.5 ml Iodessigsäureethylester zugesetzt. Nach weiteren 3 h bei 60°C wurde auf Raumtemperatur abgekühlt, mit Ether verdünnt, filtriert und das Filtrat im Vakuum eingedampft. Der Rückstand wurde durch Chromatographie an einer Kieselgelsäule (200 g) mit Ethylacetat als Eluens gereinigt. Man erhielt 1.11 g 12a, farblose Kristalle vom Fp. 85-87°C,
¹H-NMR (60 MHz, CDCl₃)
δ=1.2 (t, J=8Hz, 3H), 1.4 (t, J=8Hz, 3H), 4.2 (q, J=8Hz, 2H), 4.3 (q, J=8Hz, 2H), 5.5 (s, 2H), 5.75 (s, 2H), 8.2 (dd, J=6Hz, J'=2Hz, 1H), 8.7 (d, J=5Hz, 1H), 9.2 (s, 1H) ppm.
C₁₅H₁₈N₉O₄ Ber. 388 Gef. 388 (M⁺+H⁺)
sowie 0,87 g 12b, farblose Kristalle vom Fp. 143-144°C,
¹H-NMR (60 MHz, CDCl₃)
δ=1.3 (t, J=8Hz, 3H), 1.35 (t, J=8Hz, 3H), 4.25 (q, J=8Hz, 2H), 4.3 (q, J=8Hz, 2H), 5.5 (s, 4H), 8.2 (dd, J=6Hz, J'=2Hz, 1H), 9.0 (sh, 2H) ppm.
C₁₅H₁₈N₉O₄ Ber. 388 Gef. 388 (M⁺+H⁺)

### 2,4-Bis-[2-(2-morpholinoethyl)-tetrazol-5-yl]-pyridin (13)

Eine Lösung von 0,5 g 2,4-Bis-(tetrazol-5-yl)-pyridin, 1.2 ml Triethylamin und 0.8 g N-(2-chloracetyl)-morpholin-hydrochlorid in 5 ml abs. DMF wurde während 5 Tagen bei Raumtemperatur gerührt, anschließend mit Ether verdünnt, filtriert und das Filtrat im Vakuum eingedampft. Der Rückstand wurde durch Chromatographie an einer Kieselgelsäule (100 g) mit CH₂Cl₂/Methanol 40:1 als Eluens gereinigt. Man erhielt 214 g 13 als farbloses, zähes Öl.
¹H-NMR (60 MHz, CDCl₃)
δ=2,1 (t, J=5Hz, 4H), 2.2 (t, J=5Hz, 4H), 2.9 (t, J=6Hz, 2H), 3.1 (t, J=6Hz, 2H), 3.5 (t, J=5Hz, 4H), 3.7 (t, J=5Hz, 4H), 4.9 (t, J=6Hz, 2H), 5.2 (J=5Hz, 2H), 8.2 (dd, J=6Hz, J'=2Hz, 1H), 8.9 (d, J=6Hz, 1H, 9.1 (s, 1H) ppm.
C₁₉H₂₆N₁₁O₂ Ber. 442 Gef. 442 (M⁺+H⁺)

### 2,4-Bis-(2-benzyloxymethyltetrazol-5-yl)-pyridin (14)

Zu einer Lösung von 1.5 g 2,4-Bis-(tetrazol-5-yl)-pyridin und 1,4 ml Triethylamin in 7 ml abs. DMF wurden mit der Spritze 2.8 ml Benzylchlormethylether zugetropft und das Gemisch während 2 h bei Raumtemperatur gerührt. Anschließend wurde mit Ethanol versetzt, filtriert und das Filtrat im Vakuum eingedampft. Der Rückstand wurde an einer Kieselgelsäule (200 g) mit Ethylacetat/Heptan 1:1 als Eluens gereinigt. Man erhielt 0.56 g 14, farbloses, zähes Öl. Ein sehr reines Produkt wurde durch nachfolgende Chromatographie an RP₁₈-Kieselgel mit H₂O/Acetonitril 1:3 als Eluens erhalten.
¹H-NMR (60 MHz, CDCl₃)
δ=4.8 (s, 4H), 6.1 (2s, 4H), 7.4 (2s, 12H), 8.2 (dd, J=6Hz, J'=2Hz, 1H), 9.0 (sh, 2H) ppm.
C₂₃H₂₂N₉O₂ Ber. 456 Gef. 456 (M⁺+H⁺).

### Isomere 2,5-Bis-(benzyloxymethyltetrazol-5-yl)-pyridine (15a-d)

Analog obiger Vorschrift wurden 1.5 g 2,5-Bis-(tetrazol-5-yl)-pyridin mit 2.8 ml Benzylchlormethylether umgesetzt. Chromatographie des Rohprodukts an Kieselgel (200 g) mit Heptan/Ethylacetat 1:1 als Eluens lieferte alle isomeren Substitutionsprodukte. In der Reihenfolge der Elution erhielt man:
15a, farblose Nadeln, Fp. 103-104°C (Heptan/Ethylacetat)
¹H-NMR (CDCl₃, 60 MHZ)
δ=4.75 (s, 2H), 4.80 (s, 2H), 6.1 (s, 2H), 6.5 (s, 2H), 7.35 (s, 5H), 7.40 (s, 5H), 8.7 (sh, 2H), 9.6 (bs, 1H) ppm.
C₂₃H₂₂N₉O₂ Ber. 456 Gef. 456 (M⁺+H⁺)
15b, farbloses Kristallpulver, Fp. 137.5-138.5°C
   (Heptan/Ethylacetat),
¹H-NMR (CDCl₃, 60 MHz)
δ=4.75 (s, 4H), 6.05 und 6.10 (s, je 2H), 7.5 (s, 10H), 8.6 (m, 2H), 9.65 (bs, 1H) ppm.
C₂₃H₂₂N₉O₂ Ber. 456 Gef. 456 (M⁺+H⁺)
15c, farblose, feine Nadeln, Fp. 132-133°C (Ethylacetat/Heptan),
¹H-NMR (CDCl₃, 60MHz)
δ=4.7 (s, 2H), 4.8 (s, 2H), 5.9 (s, 2H), 6.5 (s, 2H), 7.3 (s, 5H), 7.4 (s, 5H), 8.6 (d, J=Hz, 2H), 9.4 (s, 1H) ppm.
C₂₃H₂₂N₉O₂ Ber. 456 Gef. 456 (M⁺+H⁺)
15d, farblose, weiche Nadeln, Fp. 120.5-121°C
   (Heptan/Ethylacetat),
¹H-NMR (CDCl₃, 60 MHz)
δ=4.7 (s, 4H), 5.9 (s, 2H), 6.1 (s, 2H), 7.4 (s, 10H), 8.55 (s, 2H), 9.5 (bs, 1H) ppm.
C₂₃H₂₂N₉O₂ Ber. 456 Gef. 456 (M⁺+H⁺)

## Patentansprüche (Patentansprüche für folgende(n) Vertragsstaat(en): AT, BE, CH, LI, DE, DK, FR, GB, IT, LU, NL, PT, SE)

1. Verbindungen der allgemeinen Formel I 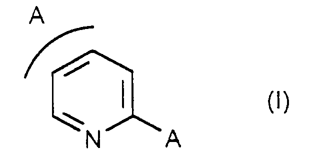 wobei A 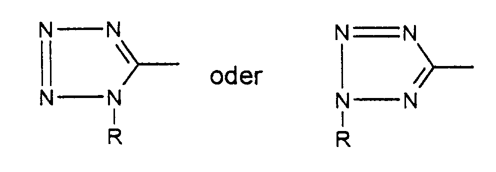 bedeutet und hierunter
R H,
(C₁-C₆)Alkyl, (C₂-C₆)Alkenyl, (C₂-C₆)Alkinyl, jeweils gegebenenfalls substituiert durch Carboxyl oder Carboxy(C₁-C₄)alkylester, durch Phenylcarbonyl, durch (C₁-C₄)Alkylaminocarbonyl, wobei Alkyl seinerseits durch (C₁-C₆)-Alkoxy substituiert sein kann, durch Aminocarbonyl, wobei der Stickstoff einfach oder zweifach mit (C₁-C₃)Alkyl substituiert sein kann,
(C₆-C₁₀)Aryl, gegebenenfalls substituiert durch Halogen,
(C₁-C₆)Alkyl oder (C₁-C₃)Alkoxy,
Phenyl(C₂-C₆)-alkyl, wobei gegebenenfalls 1-3 CH₂-Gruppen durch Heteroatome N, O oder S ersetzt sind,
(C₅-C₆)Cycloalkyl- oder (C₅-C₆)Cycloalkenyl (Cₒ-C₆)-alkyl, gegebenenfalls mit ankondensierten Benzolring, wobei 1 - 3 CH₂-Gruppen des Cyclus durch Heteroatome O, S oder N oder C=O ersetzt sind, und der Cyclus gegebenenfalls durch (C₁-C₄)Alkyl substituiert sein kann
sowie deren physiologisch verträglichen Salze.

2. Verbindungen nach Anspruch 1, dadurch gekennzeichnet, daß
R H,
(C₁-C₆)Alkyl, (C₂-C₆)Alkenyl, (C₂-C₆)Alkinyl, jeweils substituiert durch Carboxyl oder Carboxy(C₁-C₄)alkylester, durch Phenylcarbonyl, durch (C₁-C₄)Akylaminocarbonyl, durch Carbonyl, Aminocarbonyl, bedeutet.

3. Verbindungen nach den Ansprüchen 1 oder 2, dadurch gekennzeichnet, daß
R (C₁-C₆)Alkyl, (C₂-C₆)Alkenyl, (C₂-C₆)Alkinyl, substituiert durch Phenylcarbonyl, durch (C₁-C₄)Alkylaminocarbonyl, wobei (C₁-C₆)Alkyl seinerseits mit (C₁-C₆)Alkoxy substituiert ist, durch Aminocarbonyl, wobei der Stickstoff einfach oder zweifach mit (C₁-C₃)Alkyl substituiert ist.

4. Verbindungen nach Anspruch 1, dadurch gekennzeichnet, daß
R (C₆-C₁₀)Aryl, gegebenenfalls substituiert durch Halogen (Chlor oder Brom) bedeutet.

5. Verbindungen nach Anspruch 1, dadurch gekennzeichnet, daß
R Phenyl(C₂-C₆)alkyl, wobei eine oder zwei CH₂-Gruppen des Alkylrests durch Heteroatome ausgewählt aus N, O oder S ersetzt ist, bedeutet.

6. Verbindungen nach Anspruch 1, dadurch gekennzeichnet, daß
R (C₅-C₆)Cycloalkyl- oder (C₅-C₆)Cycloalkenyl-(C₀-C₆)alkyl, bedeutet und der Cyclus durch (C₁-C₄)Alkyl substituiert ist.

7. Verbindungen nach den Ansprüchen 1 oder 6, dadurch gekennzeichnet, daß die Verbindungen, in denen
R (C₅-C₆)Cycloalkyl- oder (C₅-C₆)Cycloalkenyl-(C₀-C₆)alkyl, bedeutet, achsensymmetrisch sind.

8. Verbindungen nach einem oder mehreren der Ansprüche 1 bis 7, dadurch gekennzeichnet, daß die Reste R für beide Substituenten identisch sind.

9. Verbindungen nach Anspruch 8, dadurch gekennzeichnet, daß die Substitutionsmuster für A (2,4 oder 2,5) identisch sind.

10. Verfahren zur Herstellung der Verbindungen gemäß den Ansprüchen 1 bis 9, dadurch gekennzeichnet, daß man eine Verbindung der Formel II 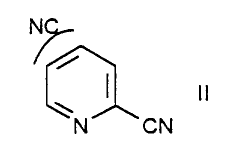 mit NaN₃ und NH₄Cl in einem geeigneten organischen Lösungsmittel umsetzt und anschließend gegebenenfalls gewünschte Substituenten in einer Substitutionsreaktion einführt.

11. Verbindungen nach den Ansprüchen 1 bis 10 zur Inhibierung der Prolin- und Lysinhydroxylase.

12. Verbindungen nach den Ansprüchen 1 bis 10 zur Anwendung als Fibrosuppressiva und Immunsuppressiva.

13. Arzneimittel, enthaltend eine Verbindung nach Anspruch 1 und/oder deren physiologisch verträglichen Salze zur Behandlung von Störungen der Biosynthese von Kollagen und kollagenähnlichen Stoffen bzw. der Biosynthese von C1_{q}.

14. Verfahren zur Herstellung von Arzneimitteln zur Beeinflussung der Biosynthese von Kollagen und kollagenähnlichen Stoffen bzw. der Biosynthese von C1_{q}, dadurch gekennzeichnet, daß man in das Arzneimittel eine Verbindung der Formel I nach Anspruch 1 und/oder ein physiologisch verträgliches Salz dieser Verbindung einverleibt.

## Patentansprüche (Patentansprüche für folgende(n) Vertragsstaat(en): ES, GR)

1. Verbindungen zur Herstellung der Verbindungen gemäß der allgemeinen Formel I 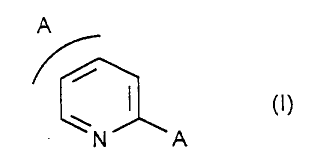 wobei A 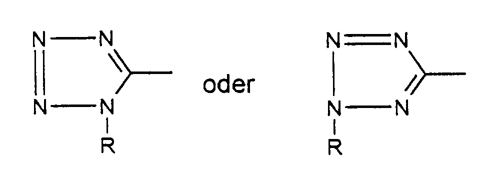 bedeutet und hierunter
R H,
(C₁-C₆)Alkyl, (C₂-C₆)Alkenyl, (C₂-C₆)Alkinyl, jeweils gegebenenfalls substituiert durch Carboxyl oder Carboxy(C₁-C₄)alkylester, durch Phenylcarbonyl, durch (C₁-C₄)Alkylaminocarbonyl, wobei Alkyl seinerseits durch (C₁-C₆)-Alkoxy substituiert sein kann, durch Aminocarbonyl, wobei der Stickstoff einfach oder zweifach mit (C₁-C₃)Alkyl substituiert sein kann,
(C₆-C₁₀)Aryl, gegebenenfalls substituiert durch Halogen,
(C₁-C₆)Alkyl oder (C₁-C₃)Alkoxy,
Phenyl(C₂-C₆)-alkyl, wobei gegebenenfalls 1-3 CH₂-Gruppen durch Heteroatome N, O oder S ersetzt sind (C₅-C₆)Cycloalkyl oder (C₅-C₆)Cycloalkeny (Cₒ-C₆)-alkyl, gegebenenfalls mit ankondensierten Benzolring, wobei 1 - 3 CH₂-Gruppen des Cyclus durch Heteroatome O, S oder N oder C=O ersetzt sind, und der Cyclus gegebenenfalls durch (C₁-C₄)Alkyl substituiert sein kann
sowie deren physiologisch verträglichen Salze. dadurch gekennzeichnet, daß man eine Verbindung der Formel II 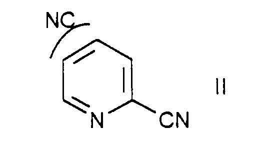 mit NaN₃ und NH₄Cl in einem geeigneten organischen Lösungsmittel umsetzt und anschließend gegebenenfalls gewünschte Substituenten in einer Substitutionsreaktion einführt.

2. Verfahren nach Anspruch 1, dadurch gekennzeichnet, daß
R H,
(C₁-C₆)Alkyl, (C₂-C₆)Alkenyl, (C₂-C₆)Alkinyl, jeweils substituiert durch Carboxyl oder Carboxy(C₁-C₄)alkylester, durch Phenylcarbonyl, durch (C₁-C₄)Alkylaminocarbonyl, durch Carbonyl, Aminocarbonyl, bedeutet.

3. Verfahren nach den Ansprüchen 1 oder 2, dadurch gekennzeichnet, daß
R (C₁-C₆)Alkyl, (C₂-C₆)Alkenyl, (C₂-C₆)Alkinyl ist, substituiert durch Phenylcarbonyl, durch (C₁-C₄)Alkylaminocarbonyl, wobei (C₁-C₆)Alkyl seinerseits mit (C₁-C₆)Alkoxy substituiert ist, durch Aminocarbonyl, wobei der Stickstoff einfach oder zweifach mit (C₁-C₃)Alkyl substituiert ist.

4. Verfahren nach Anspruch 1, dadurch gekennzeichnet, daß
R (C₆-C₁₀)Aryl, gegebenenfalls substituiert durch Halogen (Chlor oder Brom) bedeutet.

5. Verfahren nach Anspruch 1, dadurch gekennzeichnet, daß
R Phenyl(C₂-C₆)alkyl, wobei eine oder zwei CH₂-Gruppen des Alkylrests durch Heteroatome ausgewählt aus N, O oder S ersetzt ist, bedeutet.

6. Verfahren nach Anspruch 1, dadurch gekennzeichnet, daß
R (C₅-C₆)Cycloalkyl- oder (C₅-C₆)Cycloalkenyl-(C₀-C₆)alkyl, bedeutet und der Cyclus durch (C₁-C₄)Alkyl substituiert ist.

7. Verfahren nach den Ansprüchen 1 oder 6, dadurch gekennzeichnet, daß die Verbindungen, in denen
R (C₅-C₆)Cycloalkyl- oder (C₅-C₆)Cycloalkenyl-(C₀-C₆)alkyl, bedeutet, achsensymmetrisch sind.

8. Verfahren nach einem oder mehreren der Ansprüche 1 bis 7, dadurch gekennzeichnet, daß die Reste R für beide Substituenten identisch sind.

9. Verbindungen nach Anspruch 8, dadurch gekennzeichnet, daß die Substitutionsmuster für A (2,4 oder 2,5) identisch sind.

10. Verbindungen nach den Ansprüchen 1 bis 10 zur Inhibierung der Prolin- und Lysinhydroxylase.

11. Verbindungen nach den Ansprüchen 1 bis 10 zur Anwendung als Fibrosuppressiva und Immunsuppressiva.

12. Arzneimittel, enthaltend eine Verbindung nach Anspruch 1 und/oder deren physiologisch verträglichen Salze zur Behandlung von Störungen der Biosynthese von Kollagen und kollagenähnlichen Stoffen bzw. der Biosynthese von C1_{q}.

13. Verfahren zur Herstellung von Arzneimitteln zur Beeinflussung der Biosynthese von Kollagen und kollagenähnlichen Stoffen bzw. der Biosynthese von C1_{q}, dadurch gekennzeichnet, daß man in das Arzneimittel eine Verbindung der Formel I nach Anspruch 1 und/oder ein physiologisch verträgliches Salz dieser Verbindung einverleibt.

## Claims (Claims for the following Contracting State(s): AT, BE, CH, LI, DE, DK, FR, GB, IT, LU, NL, PT, SE)

1. A compound of the formula I 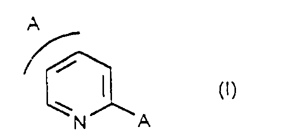 where A is 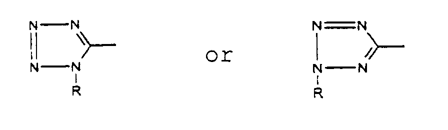 and where
R is H,
(C₁-C₆) alkyl, (C₂-C₆) alkenyl, (C₂-C₆) alkynyl, each optionally substituted by carbonyl or carboxyl (C₁-C₄) alkyl ester, by phenylcarbonyl, by (C₁-C₄)alkylaminocarbonyl, it being possible for alkyl in turn to be (C₁-C₆) alkoxy-substituted, by aminocarbonyl, it being possible for the nitrogen to be mono- or di- (C₁-C₃) alkyl-substituted,
(C₆-C₁₀) aryl, optionally substituted by halogen,
(C₁-C₆) alkyl or (C₁-C₃) alkoxy, phenyl (C₂-C₆) alkyl where 1-3 CH₂ groups are optionally replaced by hetero atoms N, O or S,
(C₅-C₆) cycloalkyl- or (C₅-C₆) cycloalkenyl (C₀-C₆) - alkyl, optionally with fused-on benzene ring, where 1-3 CH₂ groups of the cyclic system are replaced by hetero atoms O, S or N or C=0, and it being possible for the cyclic system optionally to be substituted by (C₁-C₄) alkyl, and the physiologically tolerated salts thereof.

2. A compound as claimed in claim 1, wherein
R is H,
(C₁-C₆) alkyl, (C₂-C₆) alkenyl, (C₂-C₆) alkynyl, each substituted by carboxyl or carboxy(C₁-C₄)alkyl ester, by phenylcarbonyl, by (C₁-C₄)alkyl-aminocarbonyl, by carbonyl, aminocarbonyl.

3. A compound as claimed in claim 1 or 2, wherein
R is (C₁-C₆) alkyl, (C₂-C₆) alkenyl, (C₂-C₆) alkynyl, substituted by phenylcarbonyl, by (C₁-C₄)alkyl-aminocarbonyl, where (C₁-C₆) alkyl is in turn (C₁-C₆)alkoxy-substituted by aminocarbonyl, where the nitrogen is substituted once or twice by (C₁-C₃) alkyl.

4. A compound as claimed in claim 1, wherein
R is (C₆-C₁₀)aryl, optionally substituted by halogen (chlorine or bromine).

5. A compound as claimed in claim 1, wherein
R is phenyl (C₂-C₆) alkyl, where one or two CH₂ groups in the alkyl radical is replaced by hetero atoms selected from N, O or S.

6. A compound as claimed in claim 1, wherein
R is (C₅-C₆) cycloalkyl- or (C₅-C₆) cycloalkenyl-(C₀-C₆) alkyl and the cyclic system is substituted by (C₁-C₄) alkyl.

7. A compound as claimed in claim 1 or 6, which is axially symmetrical when
R is (C₅-C₆) cycloalkyl- or (C₅-C₆) cycloalkenyl-(C₀-C₆)alkyl.

8. A compound as claimed in one or more of claims 1 to 7, wherein the radicals R are identical for both substituents.

9. A compound as claimed in claim 8, wherein the substitution patterns for A (2,4 or 2,5) are identical.

10. A process for the preparation of the compounds as claimed in claims 1 to 9, which comprises reacting a compound of the formula II 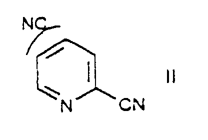 with NaN₃ and NH₄Cl in a suitable organic solvent, and subsequently where appropriate introducing required substituents in a substitution reaction.

11. A compound as claimed in claims 1 to 10 for the inhibition of proline hydroxylase and lysine hydroxylase.

12. A compound as claimed in claims 1 to 10 for use as fibrosuppressant and immunosuppressant.

13. A pharmaceutical containing a compound as claimed in claim 1 and/or the physiologically tolerated salts thereof for the treatment of disorders of the biosynthesis of collagen and collagen-like substances and of the biosynthesis of C1_{q}.

14. A process for the preparation of pharmaceuticals for influencing the biosynthesis of collagen and collagen-like substances and the biosynthesis of C1_{q}, which comprises incorporating into the pharmaceutical a compound of the formula I as claimed in claim 1 and/or a physiologically tolerated salt of this compound.

## Claims (Claims for the following Contracting State(s): ES, GR)

1. A process for the preparation of the compounds of the formula I 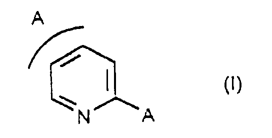 where A is 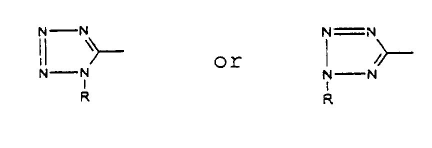 and where
R is H,
(C₁-C₆) alkyl, (C₂-C₆) alkenyl, (C₂-C₆) alkynyl, each optionally substituted by carbonyl or carboxyl (C₁-C₄) alkyl ester, by phenylcarbonyl, by (C₁-C₄) - alkylaminocarbonyl, it being possible for alkyl in turn to be (C₁-C₆) alkoxy-substituted, by aminocarbonyl, it being possible for the nitrogen to be mono- or di(C₁-C₃)alkyl-substituted,
(C₆-C₁₀)aryl, optionally substituted by halogen,
(C₁-C₆) alkyl or (C₁-C₃) alkoxy, phenyl (C₂-C₆) alkyl where 1-3 CH₂ groups are optionally replaced by hetero atoms N, O or S,
(C₅-C₆) cycloalkyl- or (C₅-C₆) cycloalkenyl (C₀-C₆) - alkyl, optionally with fused-on benzone ring, where 1-3 CH₂ groups of the cyclic system are replaced by hetero atoms O, S or N or C=0, and it being possible for the cyclic system optionally to be substituted by (C₁-C₄)alkyl, and the physiologically tolerated salts thereof, which comprises reacting a compound of the formula II 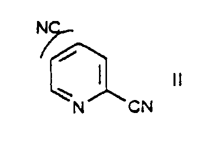 with NaN₃ and NH₄Cl in a suitable organic solvent, and subsequently where appropriate introducing required substituents in a substitution reaction.

2. The process as claimed in claim 1, wherein
R is H,
(C₁-C₆)alkyl, (C₂-C₆)alkenyl, (C₂-C₆)alkynyl, each substituted by carboxyl or carboxy (C₁-C₄) alkyl ester, by phenylcarbonyl, by (C₁-C₄) alkyl-aminocarbonyl, by carbonyl, aminocarbonyl.

3. The process as claimed in claim 1 or 2, wherein
R is (C₁-C₆)alkyl, (C₂-C₆)alkenyl, (C₂-C₆)alkynyl, substituted by phenylcarbonyl, by (C₁-C₄)alkyl-aminocarbonyl, where (C₁-C₆) alkyl is in turn (C₁-C₆) alkoxy-substituted by aminocarbonyl, where the nitrogen is substituted once or twice by (C₁-C₃) alkyl.

4. The process as claimed in claim 1, wherein
R is (C₆-C₁₀) aryl, optionally substituted by halogen (chlorine or bromine).

5. The process as claimed in claim 1, wherein
R is phenyl(C₂-C₆)alkyl, where one or two CH₂ groups in the alkyl radical is replaced by hetero atoms selected from N, O or S.

6. The process as claimed in claim 1, wherein
R is (C₅-C₆) cycloalkyl- or (C₅-C₆) cycloalkenyl-(C₀-C₆) alkyl and the cyclic system is substituted by (C₁-C₄)alkyl.

7. The process as claimed in claim 1 or 6, which is axially symmetrical when
R is (C₅-C₆) cycloalkyl- or (C₅-C₆) cycloalkenyl-(C₀-C₆) alkyl.

8. The process as claimed in one or more of claims 1 to 7, wherein the radicals R are identical for both substituents.

9. A compound as claimed in claim 8, wherein the substitution patterns for A (2,4 or 2,5) are identical.

10. A compound as claimed in claims 1 to 9 for the inhibition of proline hydroxylase and lysine hydroxylase.

11. A compound as claimed in claims 1 to 10 for use as fibrosuppressant and immunosuppressant.

12. A pharmaceutical containing a compound as claimed in claim 1 and/or the physiologically tolerated salts thereof for the treatment of disorders of the biosynthesis of collagen and collagen-like substances and of the biosynthesis of C1_{q}.

13. A process for the preparation of pharmaceuticals for influencing the biosynthesis of collagen and collagen-like substances and the biosynthesis of C1_{q}, which comprises incorporating into the pharmaceutical a compound of the formula I as claimed in claim 1 and/or a physiologically tolerated salt of this compound.

## Revendications (Revendications pour l'(les) Etat(s) contractant(s) suivant(s): AT, BE, CH, LI, DE, DK, FR, GB, IT, LU, NL, PT, SE)

1. Composés de formule générale I 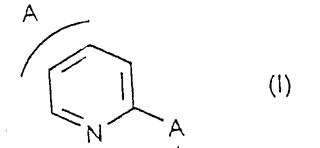 dans laquelle A représente 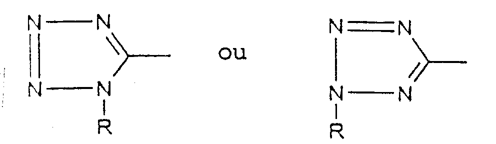 et dans ces formules
R représente H,
un groupe alkyle en C₁-C₆, alcényle en C₂-C₆, alcynyle en C₂-C₆, éventuellement substitués chacun par un groupe carboxy ou carboxylate d'alkyle en C₁-C₄, par le groupe phénylcarbonyle, par un groupe alkyl(C₁-C₄)aminocarbonyle, le fragment alkyle pouvant pour sa part être substitué par un groupe alcoxy en C₁-C₆, par un groupe aminocarbonyle, l'atome d'azote pouvant être substitué une ou deux fois par un ou des groupes alkyle en C₁-C₃, un groupe aryle en C₆-C₁₀, éventuellement substitué par un atome d'halogène ou par un groupe alkyle en C₁-C₆ ou alcoxy en C₁-C₃,
un groupe phényl-alkyle(C₂-C₆), 1-3 groupes CH₂ pouvant éventuellement être remplacés par des hétéroatomes N, O ou S,
un groupe cycloalkyl (C₅-C₆)- ou cycloalcényl (C₅-C₆)-alkyle(C₀-C₆), éventuellement avec un cycle benzénique soudé, 1 à 3 groupes CH₂ du cycle étant remplacés par des hétéroatomes O, S ou N, ou C=O, et le cycle pouvant éventuellement être substitué par un groupe alkyle en C₁-C₄,
ainsi que leurs sels physiologiquement acceptables.

2. Composés selon la revendication 1, caractérisés en ce que
R représente H,
un groupe alkyle en C₁-C₆, alcényle en C₂-C₆, alcynyle en C₂-C₆, substitués chacun par un groupe carboxy ou carboxylate d'alkyle (C₁-C₄), par le groupe phénylcarbonyle, par un groupe alkyl(C₁-C₄)aminocarbonyle, par le groupe carbonyle, aminocarbonyle.

3. Composés selon la revendication 1 ou 2, caractérisés en ce que
R représente un groupe alkyle en C₁-C₆, alcényle en C₂-C₆, alcynyle en C₂-C₆, substitués par le groupe phénylcarbonyle, par un groupe alkyl(C₁-C₄)aminocarbonyle, le fragment alkyle en C₁-C₆ pouvant pour sa part être substitué par un groupe alcoxy en C₁-C₆, par un groupe aminocarbonyle, l'atome d'azote étant une ou deux fois substitué par un ou des groupes alkyle en C₁-C₃.

4. Composés selon la revendication 1, caractérisés en ce que R représente un groupe aryle en C₆-C₁₀, éventuellement substitué par un atome d'halogène (chlore ou brome).

5. Composés selon la revendication 1, caractérisés en ce que R représente un groupe phényl-alkyle(C₂-C₆), un ou deux groupes CH₂ du fragment alkyle étant remplacés par des hétéroatomes choisis parmi N, O et S.

6. Composés selon la revendication 1, caractérisés en ce que R représente un groupe cycloalkyl(C₅-C₆)- ou cycloalcényl(C₅-C₆)-alkyle(C₀-C₆) et le cycle est substitué par un groupe alkyle en C₁-C₄.

7. Composés selon la revendication 1 ou 6, caractérisés en ce que les composés dans lesquels R représente un groupe cycloalkyl(C₅-C₆)- ou cycloalcényl(C₅-C₆)alkyle-(C₀-C₆) ont une symétrie axiale.

8. Composés selon une ou plusieurs des revendications 1 à 7, caractérisés en ce que les radicaux R sont identiques pour les deux substituants.

9. Composés selon la revendication 8, caractérisés en ce que les types de substitution pour A (2,4 ou 2,5) sont identiques.

10. Procédé pour la préparation des composés selon les revendications 1 à 9, caractérisé en ce que l'on fait réagir un composé de formule II 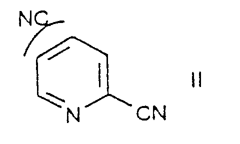 avec NaN₃ et NH₄Cl dans un solvant organique approprié, et ensuite on introduit éventuellement des substituants désirés, dans une réaction de substitution.

11. Composés selon les revendications 1 à 10, pour l'inhibition de la proline hydroxylase et de la lysine hydroxylase.

12. Composés selon les revendications 1 à 10, pour utilisation en tant que fibrosuppresseurs et immunosuppresseurs.

13. Médicaments, contenant un composé selon la revendication 1 et/ou des sels physiologiquement acceptables d'un tel composé, pour le traitement de troubles de la biosynthèse du collagène et de substances de type collagène, ou de la biosynthèse du C1_{q}.

14. Procédé pour la fabrication de médicaments destinés à influer sur la biosynthèse du collagène et de substances de type collagène ou sur la biosynthèse du C1_{q}, caractérisé en ce que l'on incorpore dans le médicament un composé de formule I selon la revendication 1 et/ou un sel physiologiquement acceptable de ce composé.

## Revendications (Revendications pour l'(les) Etat(s) contractant(s) suivant(s): ES, GR)

1. Procédé pour la préparation des composés selon la formule générale I 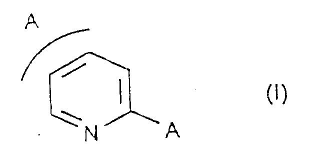 dans laquelle A représente 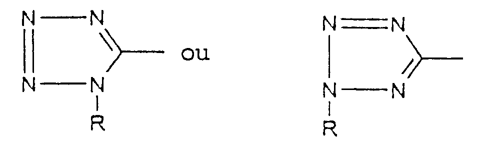 et dans ces formules
R représente H,
un groupe alkyle en C₁-C₆, alcényle en C₂-C₆, alcynyle en C₂-C₆, éventuellement substitués chacun par un groupe carboxy ou carboxylate d'alkyle en C₁-C₄, par le groupe phénylcarbonyle, par un groupe alkyl (C₁-C₄) aminocarbonyle, le fragment alkyle pouvant pour sa part être substitué par un groupe alcoxy en C₁-C₆, par un groupe aminocarbonyle, l'atome d'azote pouvant être substitué une ou deux fois par un ou des groupes alkyle en C₁-C₃, un groupe aryle en C₆-C₁₀, éventuellement substitué par un atome d'halogène ou par un groupe alkyle en C₁-C₆ ou alcoxy en C₁-C₃,
un groupe phényl-alkyle(C₂-C₆), 1-3 groupes CH₂ pouvant éventuellement être remplacés par des hétéroatomes N, O ou S,
un groupe cycloalkyl(C₅-C₆)- ou cycloalcényl(C₅-C₆)-alkyle(C₀-C₆), éventuellement avec un cycle benzénique soudé, 1 à 3 groupes CH₂ du cycle étant remplacés par des hétéroatomes O, S ou N, ou C=O, et le cycle pouvant éventuellement être substitué par un groupe alkyle en C₁-C₄,
ainsi que de leurs sels physiologiquement acceptables, caractérisé en ce que l'on fait réagir un composé de formule II 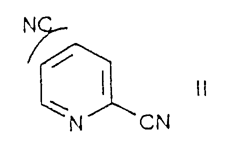 avec NaN₃ et NH₄Cl dans un solvant organique approprié, et ensuite on introduit éventuellement des substituants désirés, dans une réaction de substitution.

2. Procédé selon la revendication 1, caractérisé en ce que
R représente H,
un groupe alkyle en C₁-C₆, alcényle en C₂-C₆, alcynyle en C₂-C₆, substitués chacun par un groupe carboxy ou carboxylate d'alkyle(C₁-C₄), par le groupe phénylcarbonyle, par un groupe alkyl(C₁-C₄)aminocarbonyle, par le groupe carbonyle, aminocarbonyle.

3. Procédé selon la revendication 1 ou 2, caractérisé en ce que
R représente un groupe alkyle en C₁-C₆, alcényle en C₂-C₆, alcynyle en C₂-C₆, substitués par le groupe phénylcarbonyle, par un groupe alkyl(C₁-C₄)aminocarbonyle, le fragment alkyle en C₁-C₆ pouvant pour sa part être substitué par un groupe alcoxy en C₁-C₆, par un groupe aminocarbonyle, l'atome d'azote étant une ou deux fois substitué par un ou des groupes alkyle en C₁-C₃.

4. Procédé selon la revendication 1, caractérisé en ce que R représente un groupe aryle en C₆-C₁₀, éventuellement substitué par un atome d'halogène (chlore ou brome).

5. Procédé selon la revendication 1, caractérisé en ce que R représente un groupe phényl-alkyle(C₂-C₆), un ou deux groupes CH₂ du fragment alkyle étant remplacés par des hétéroatomes choisis parmi N, O et S.

6. Procédé selon la revendication 1, caractérisé en ce que R représente un groupe cycloalkyl(C₅-C₆)- ou cycloalcényl(C₅-C₆)-alkyle(C₀-C₆) et le cycle est substitué par un groupe alkyle en C₁-C₄.

7. Procédé selon la revendication 1 ou 6, caractérisé en ce que les composés dans lesquels R représente un groupe cycloalkyl(C₅-C₆)- ou cycloalcényl(C₅-C₆)alkyle-(C₀-C₆) ont une symétrie axiale.

8. Procédé selon une ou plusieurs des revendications 1 à 7, caractérisé en ce que les radicaux R sont identiques pour les deux substituants.

9. Procédé selon la revendication 8, caractérisés en ce que les types de substitution pour A (2,4 ou 2,5) sont identiques.

10. Composés selon les revendications 1 à 10, pour l'inhibition de la proline hydroxylase et de la lysine hydroxylase.

11. Composés selon les revendications 1 à 10, pour utilisation en tant que fibrosuppresseurs et immunosuppresseurs.

12. Médicaments, contenant un composé selon la revendication 1 et/ou des sels physiologiquement acceptables d'un tel composé, pour le traitement de troubles de la biosynthèse du collagène et de substances de type collagène, ou de la biosynthèse du C1_{q}.

13. Procédé pour la fabrication de médicaments destinés à influer sur la biosynthèse du collagène et de substances de type collagène ou sur la biosynthèse du C1_{q}, caractérisé en ce que l'on incorpore dans le médicament un composé de formule I selon la revendication 1 et/ou un sel physiologiquement acceptable de ce compose.
